# FASCICULE DE BREVET EUROPEEN

(11) **EP 2 384 784 B1**
(45) Date de publication et mention de la délivrance du brevet: **26.09.2012**
(21) Numéro de dépôt: 11153961.5
(22) Date de dépôt: 10.02.2011
(51) Int. Cl.: A61N 1/05, A61N 1/362, A61N 1/375

(54) **Ensemble de stimulation/défibrillation endocavitaire du ventricule gauche**
Einheit zur endokavitären Stimulation/Defibrillation des linken Herzventrikels
Assembly for endocavitary stimulation/defibrillation of the left ventricle

(30) Priorité: 05.05.2010 FR 1053499
(43) Date de publication de la demande: 09.11.2011
(73) Titulaire: Sorin CRM SAS, 92143 Clamart Cedex (FR)
(72) Inventeur: Ollivier, Jean-François, 91190 Villiers le Bacle (FR)
(74) Mandataire: Dupuis-Latour, Dominique

(56) Documents cités:
- WO-A2-2008/058265
- US-A- 5 728 140
- US-A1- 2010 010 607

## Description

L'invention concerne la stimulation du ventricule gauche sur un site endocavitaire, par application directe d'impulsions sur la paroi interne de ce ventricule.

L'invention se situe dans le contexte général des "dispositifs médicaux implantables actifs" tels que définis par la directive 90/385/CEE du 20 juin 1990 du Conseil des communautés européennes, notamment les implants permettant de surveiller en continu le rythme cardiaque et délivrer si nécessaire au coeur des impulsions électriques de stimulation, de resynchronisation ou de défibrillation.

On fera principalement référence dans la présente description à des sondes endocavitaires "de stimulation", c'est-à-dire destinées à la délivrance d'impulsions à basse énergie utilisées pour les thérapies antibradycardiques ou de resynchronisation. Mais l'invention s'applique également aux sondes endocavitaires de cardioversion/défibrillation destinées à délivrer au coeur des chocs électriques de haute énergie pour tenter de mettre fin à une tachyarythmie. Sauf indication contraire, les termes génériques de "sonde (ou électrode) de stimulation", ou "de stimulation/défibrillation" viseront tout type de sonde utilisable à ces fins.

Pour la stimulation du ventricule droit, il suffit d'implanter une sonde endocavitaire par le réseau veineux périphérique droit. En revanche, pour stimuler le ventricule gauche, la situation est plus complexe et la solution la plus souvent retenue consiste à introduire la sonde dans le réseau coronarien via l'oreillette droite puis l'ostium du sinus coronaire.

Une telle sonde - qui peut être qualifiée de sonde endocardiaque mais non de sonde endocavitaire - est décrite par exemple dans le EP 0 993 840 A1 (ELA Médical), correspondant au produit commercialisé sous la dénomination *Situs LV* par Sorin CRM, Clamart, France.

Cette technique d'implantation n'est cependant pas toujours réalisable, notamment lorsque la conformation du sinus coronaire est trop accidentée, ou en cas de thrombose. En effet, le positionnement précis de l'électrode ou des électrodes destinée(s) à stimuler le ventricule gauche au travers de la paroi du myocarde est un paramètre critique, et une configuration satisfaisante du ou des sites de stimulation n'est pas toujours possible à obtenir.

Une autre technique consiste à stimuler le ventricule gauche en appliquant des impulsions de stimulation à la paroi du septum ventriculaire (c'est-à-dire la cloison interventriculaire), au moyen d'une sonde introduite dans le ventricule droit. Cette technique de stimulation du ventricule gauche par une sonde introduite dans le ventricule droit est décrite par exemple dans le EP 1 428 550 A1 (De Bellis/P.A.& M. SpA).

Le US 5 728 140 A décrit également une telle technique de stimulation transseptale du ventricule gauche, sans introduction d'électrode dans la cavité gauche. Plus précisément, ce dispositif prévoit d'implanter dans le septum une électrode à vis pénétrant la paroi droite du septum, mais dont la longueur est légèrement inférieure à l'épaisseur de la paroi à l'endroit du site de stimulation, de manière que l'extrémité de la vis approche la paroi septale gauche sans la traverser.

Si l'on souhaite stimuler directement le ventricule gauche par voie endocavitaire, les seules techniques proposées jusqu'à présent consistent à percer le septum interauriculaire ou interventriculaire, puis à introduire une sonde traversant ce septum jusqu'à venir en contact avec un point de la paroi du ventricule gauche, où elle sera ancrée (par exemple par une vis de fixation). Les impulsions de stimulation seront appliquées directement au site endocavitaire gauche ainsi choisi.

Cette procédure, telle qu'elle est mise en oeuvre actuellement, présente cependant des risques opératoires élevés, notamment des risques de perforation accidentelle de l'aorte, ou encore de dissection des parois de l'oreillette droite par un mouvement rotatoire inopiné de l'aiguille de perçage du septum.

Un autre risque de cette technique est celui d'une embolie gazeuse, du fait que l'élément débouchant dans le ventricule gauche est un cathéter creux. Pour éviter ce risque, il est impératif de prendre de nombreuses précautions lors du maniement des valves hémostatiques, de respecter des procédures de purge du matériel, etc. Toutefois, compte tenu du caractère très invasif de cette procédure, des incertitudes demeurent sur le comportement du dispositif à long terme dans la circulation artérielle, ce qui implique un traitement anticoagulant pour éviter tout risque thrombo-embolique postopératoire.

Enfin, cette technique exclut pratiquement toute extraction ultérieure de la sonde, compte tenu des risques trop importants qui seraient encourus au niveau de la traversée du septum.

En tout état de cause, cette technique est très délicate à mettre en oeuvre et requiert une grande habileté du praticien qui doit, pour pouvoir traverser le septum, s'assurer toujours du positionnement parfait de l'aiguille de perçage sur la paroi du septum, la traversée du septum ne devant être entreprise que s'il ne subsiste aucun doute sur la position de l'aiguille. Des nécessaires de perçage spécifiques ont d'ailleurs été développés à cet effet, par exemple celui décrit dans le EP 1 516 644 A1 (ELA Medical).

Les inconvénients exposés ci-dessus sont ceux que l'on rencontrerait notamment à la mise en oeuvre d'un ensemble tel que celui décrit par le WO 2008/058265 A2.

Ce document propose d'utiliser comme "électrode ventriculaire gauche" un élément, introduit dans un cathéter à vis, comprenant une extrémité pointue et pourvue d'un organe en forme de harpon destiné à assurer un ancrage de la pointe à la paroi de la cavité gauche. Cette électrode est sollicitée au moyen d'un poussoir introduit dans le cathéter, de manière à enfoncer la pointe au travers du septum puis déployer l'électrode dans le ventricule gauche. Le cathéter est ensuite vissé à la paroi du septum, côté droit.

L'ensemble décrit par ce document nécessite des manoeuvres très délicates pour sa pose, très éloignées de ce dont les praticiens ont l'habitude. Son utilisation est en outre très traumatique pour les tissus, tant du septum que de la cavité gauche, compte tenu du diamètre nécessairement élevé des trois éléments imbriqués télescopiquement les uns dans les autres (cathéter, électrode déployable et poussoir). La pose n'est bien entendu pas réversible, en raison de la pointe en forme de harpon et du diamètre important du perçage réalisé dans le septum, qui ne pourrait pas se refermer naturellement en cas de retrait. De plus, la surface de stimulation est relativement élevée, ce qui conduit à de mauvaises performances électriques et à une consommation élevé de courant.

Le but de l'invention est de proposer un nouveau type d'ensemble de stimulation endocavitaire du ventricule gauche du type précité tel que divulgué par le WO 2008/058265 - c'est-à-dire avec implantation d'une sonde directement dans la cavité ventriculaire gauche après traversée du septum - qui soit beaucoup moins invasif et pallie les multiples inconvénients précités des techniques actuelles.

Le point de départ de l'invention réside dans la constatation de ce que ces inconvénients sont essentiellement dus au fait que, dans les techniques proposées jusqu'à présent, il est nécessaire de réaliser dans la paroi septale une ponction de diamètre suffisante pour pouvoir y introduire un cathéter-guide. Ce cathéter-guide est destiné à établir une communication entre ventricule droit et ventricule gauche au travers de la paroi du septum, afin de pouvoir y introduire ensuite la sonde de stimulation endocavitaire gauche.

L'idée de base de l'invention consiste à supprimer ce cathéter-guide associé à une sonde traversant le septum, en remplaçant cet ensemble par une sonde de type conventionnel vissée sur la paroi du ventricule droit au niveau du septum, et en prolongeant cette sonde par un microcâble transseptal, partiellement isolé et poussé dans le ventricule gauche jusqu'à venir en contact contre une cible située dans ce ventricule, par exemple contre la paroi libre de ce dernier.

Plus précisément, l'ensemble de l'invention est un ensemble du type général divulgué par le WO 2008/058265 A2, comprenant, de manière en elle-même connue : un boîtier de stimulateur, défibrillateur et/ou resynchroniseur implantable comprenant des moyens générateurs aptes à délivrer entre deux bornes des impulsions électriques de stimulation/défibrillation ; et une sonde endocavitaire comprenant côté distal au moins une électrodes de stimulation/défibrillation couplées côté proximal à une borne correspondante des moyens générateurs.

La sonde comprend un corps de sonde en matériau déformable avec une lumière interne ouverte côté distal, l'extrémité distale du corps de sonde étant apte à s'étendre dans la cavité du ventricule droit et étant pourvue de moyens d'ancrage à la paroi du septum interventriculaire, le corps de sonde portant au moins une électrode de stimulation/défibrillation couplée côté proximal à une première des deux bornes des moyens générateurs. La sonde comprend également : au moins une structure apte à être déployée dans la cavité du ventricule gauche, comprenant un coeur électriquement conducteur protégé par une gaine isolante, cette structure étant logée et apte à coulisser dans la lumière du corps de sonde en s'étendant sur toute la longueur du corps de sonde et au-delà de l'extrémité distale de celui-ci. La partie de la structure située au-delà de l'extrémité distale du corps de sonde comprend successivement : une partie intermédiaire apte à traverser de part en part la paroi du septum interventriculaire, et une partie libre active apte à émerger dans la cavité du ventricule gauche, cette partie libre comprenant au moins une région dénudée. La structure est électriquement couplé à son extrémité proximale à la seconde des deux bornes des moyens générateurs, de manière à produire lors de l'activation de ces moyens générateurs un champ électrique entre, d'une part, la (les) électrode(s) de stimulation/défibrillation du corps de sonde et, d'autre part, la (les) région(s) dénudée(s) de la partie libre active du microcâble.

De façon caractéristique de l'invention, ladite structure apte à être déployée dans la cavité du ventricule gauche est un microcâble présentant un diamètre de coeur électriquement conducteur compris, au moins dans la partie libre active, entre 0,2 et 0,4 mm. Ce microcâble comporte un toron formé d'une pluralité de brins avec au moins un brin composite comprenant un premier matériau présentant des propriétés de résistance mécanique à la fatigue en flexion supérieures à celles des autres matériaux du brin, et un deuxième matériau radio-opaque.

Selon diverses caractéristiques subsidiaires avantageuses :
- la longueur de la partie libre active est comprise entre 30 et 120 mm, de préférence 45 à 55 mm, et la longueur de la partie intermédiaire est comprise entre 10 et 20 mm ;
- le diamètre de la gaine isolante du microcâble est compris entre 0,5 et 0,7 mm ;
- ledit au moins un brin composite comprend en outre un troisième matériau présentant des propriétés de conductivité électrique supérieures à celles des autres matériaux du brin ;
- le toron du microcâble est formé d'une pluralité de brins composites, avec un fil d'âme comprenant ledit deuxième matériau, ce fil d'âme étant entouré par des fils composites comprenant chacun ledit premier matériau comme matériau de surface ;
- la partie libre active du microcâble comporte une pluralité de régions dénudées distinctes successives, ou bien une région dénudée hélicoïdale, s'étendant le long de cette partie libre active ;
- la partie libre active du microcâble est pourvue, à l'exception de l'extrémité distale, d'un manchon raidisseur apte à faire varier la raideur de l'ensemble le long de la partie émergente, avantageusement un tube creux formé de brins hélicoïdaux de flexibilité contrôlée et partiellement revêtu d'un isolant, de manière à définir une structure bipolaire ou multipolaire ;
- la surface totale de la (des) région(s) dénudée(s) de la partie libre active du microcâble est d'au plus 40 mm², de préférence comprise entre 4 et 6 mm²;
- l'ensemble comprend également un générateur de ponction radiofréquence, apte à être relié au microcâble pour permettre l'application contrôlée à l'extrémité distale de celui-ci d'une énergie radiofréquence pour réaliser une traversée de la paroi du septum par ce microcâble ;
- les moyens d'ancrage de l'extrémité distale du corps de sonde comprennent une vis hélicoïdale saillante apte à pénétrer dans la paroi du septum interventriculaire sous l'effet d'un mouvement de vissage imprimé au corps de sonde depuis l'extrémité proximale celui-ci ;
- l'électrode du corps de sonde couplée à ladite première borne des moyens générateurs peut être une électrode de stimulation, disposée dans la région distale du corps de sonde, ou bien un bobinage formant électrode de défibrillation, de type RV apte à être disposé dans le ventricule droit ou de type SVC apte à être disposé dans la veine cave supérieure au voisinage de l'oreillette ;
- la partie libre active du microcâble comprend une partie d'extrémité distale présentant une raideur en flexion inférieure à celle d'une partie attenante moins distale ;
- la partie libre active est comprise entre 5 et 15 mm et est conformée en une boucle plate.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
Les Figures 1 à 3 illustrent les différentes étapes de la mise en place d'un ensemble de stimulation endocavitaire du ventricule gauche selon l'invention.
La Figure 4 est homologue de la Figure 3, dans une variante de mise en oeuvre de l'invention.
La Figure 5 est une vue de détail de la région de traversée du septum interventriculaire dans la configuration finale de la Figure 3 ou de la Figure 4.
La Figure 6 est une coupe transversale fortement agrandie du microcâble, montrant la structure interne de celui-ci.
La Figure 7 illustre un mode de mise en oeuvre dans lequel la paroi émergente du microcâble comporte une région dénudée en forme de rainure hélicoïdale.
La Figure 8 illustre une autre variante de réalisation de la partie émergente du microcâble, présentant une structure multipolaire, une rigidité en flexion variable et une pluralité de régions dénudées discrètes.

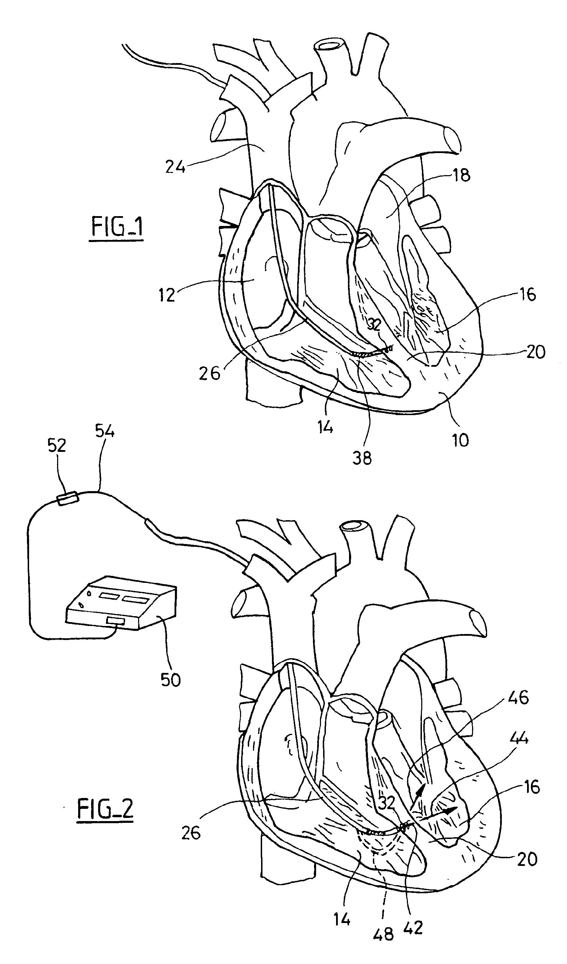

On va maintenant décrire un exemple de mise en oeuvre de l'invention. Sur la Figure 1, la référence 10 désigne de façon générale le muscle cardiaque, montrant l'oreillette droite 12, le ventricule droit 14, le ventricule gauche 16 et l'oreillette gauche 18. Les deux ventricules 14 et 16 sont séparés par la cloison ou septum interventriculaire 20, qui présente une épaisseur de l'ordre de 10 à 15 mm. Les deux oreillettes 12 et 18, quant à elles, sont séparées par le septum interauriculaire, qui présente une épaisseur de 2 à 3 mm.

La Figure 1 illustre la première étape de l'intervention, qui consiste à introduire - par une approche en elle-même conventionnelle - une sonde 26 de type sonde à vis dans le ventricule droit 14 via la veine cave supérieure 24 et à ancrer cette sonde dans la paroi du septum interventriculaire 20.

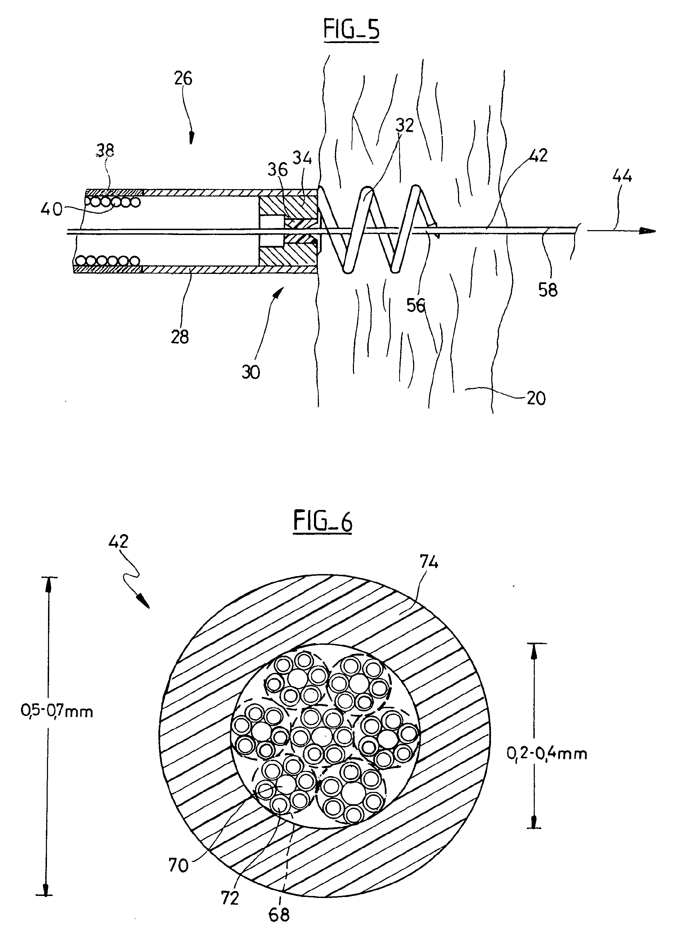

Cette sonde est par exemple une sonde de type *Stelid BS46D ou Beflex RF46D* commercialisées par Sorin CRM, Clamart, France, comportant un corps de sonde de structure conventionnelle, avec une gaine 28 en matériau déformable, généralement une gaine en polyuréthanne ou silicone. Le corps de sonde est terminé à son extrémité distale, représentée plus en détail Figure 5, par une tête de sonde 30 comprenant une vis hélicoïdale 32 à spires non jointives dont le diamètre est par exemple de l'ordre de 1 à 2 mm. Cette vis est solidarisée à la tête de sonde par un embout 34 comportant en son centre une lumière et des moyens d'étanchéité 36, par exemple un bouchon percé en silicone, permettant d'éviter tout reflux de sang à l'intérieur du corps de sonde aussi bien en l'absence qu'en présence d'un élément introduit dans la lumière centrale de l'embout 34 (comme dans le cas illustré Figure 5).

La sonde porte également une électrode distale 38 électriquement reliée à un conducteur interne 40, qui est par exemple un conducteur spiralé s'étendant sur toute la longueur de la gaine jusqu'à l'extrémité opposée, proximale, de la sonde, extrémité terminée par un connecteur électrique (non représenté) destiné à être couplé au boîtier du générateur implanté tel qu'un stimulateur/défibrillateur ou un resynchroniseur.

Le matériau et les dimensions de la gaine 28 peuvent être choisis, en combinaison avec le conducteur spiralé 40, pour procurer une certaine rigidité en torsion du corps de sonde, de manière à pouvoir transmettre un couple de torsion depuis l'extrémité proximale de la sonde (au niveau du connecteur électrique) jusqu'à l'extrémité distale 30 de la tête de sonde. Cette rotation doit permettre d'entraîner en rotation la vis 32 pour la faire pénétrer par vissage dans le tissu cardiaque.

La pose de la sonde 26 est réalisée en suivant le mode opératoire ci-après.

La première phase consiste à repérer le site d'ancrage, en manipulant l'extrémité de la sonde 26 par l'intermédiaire d'un mandrin classique introduit dans la lumière de celle-ci. L'ensemble est introduit dans la veine cave supérieure 24, l'oreillette droite 12 puis le ventricule 14 jusqu'à venir en butée contre la paroi 20 du septum interventriculaire.

Une fois ce site atteint, le praticien imprime une rotation axiale au corps de sonde, ce qui va avoir pour effet de faire pénétrer la vis hélicoïdale 32 dans la paroi du septum interventriculaire 20, le vissage complet étant détecté tactilement par le praticien du fait de la résistance opposée à la rotation. Le mouvement de rotation axiale est imprimé, selon le cas, soit directement au corps de sonde (gaine 28 et conducteur spiralé 40), soit à la fiche du connecteur pour une sonde de type *pin driven* (dans le cas où, côté proximal, la fiche de connexion est solidaire d'un conducteur axial s'étendant à l'intérieur du corps de sonde, ce conducteur étant lui-même libre en rotation et relié à la vis hélicoïdale à son extrémité distale).

On notera que dans le cas d'une vis rétractable (au moyen d'un mécanisme connu), le mouvement de rotation axiale déploie simultanément la vis hors de son logement et assure sa pénétration dans la paroi du myocarde.

En variante ou en complément, si la rigidité en torsion du corps de sonde n'est pas suffisante, il est possible d'utiliser un mandrin de vissage spécifique introduit dans la lumière interne de la sonde, l'extrémité de ce mandrin étant couplée à l'embout 34 pour permettre l'entraînement en rotation de ce dernier, et donc de la vis 32, directement depuis l'extrémité proximale de la sonde.

Le site est ensuite confirmé par examen radiographique selon différentes inclinaisons ; si la position n'est pas satisfaisante le praticien peut dévisser la tête de sonde et déplacer celle-ci sous contrôle vers un autre point, puis tester le nouveau site.

Une sonde à vis implantée telle que la sonde 26 que l'on vient de décrire est généralement utilisée comme sonde de détection/stimulation après ancrage de la vis au site de stimulation endocavitaire.

Dans le cas de l'invention, cette sonde sera utilisée non seulement comme support d'au moins une électrode de détection/stimulation (l'électrode distale 38 dans l'exemple illustré), mais également comme outil de guidage d'un microcâble au travers de la paroi du septum et au-delà de celle-ci.

Comme illustré Figures 2 et 5, le microcâble 42 est introduit dans la lumière centrale de la gaine 28 du corps de sonde jusqu'à l'embout 34 et au travers du bouchon silicone percé 36, jusqu'à venir en butée contre la paroi septale 20, en étant orienté sensiblement dans la direction 44 de l'axe central de la vis 32.

Le praticien peut contrôler l'orientation de la direction axiale 44 de progression du microcâble 42 en jouant sur la courbure de la sonde 26 dans la région voisine de la vis d'ancrage 32. Plus précisément, l'orientation du microcâble peut être déplacée vers le haut du ventricule gauche, comme en 46, en poussant légèrement la sonde 26 de manière à accentuer la courbure à proximité de la vis d'ancrage, comme illustré en tiretés en 48 Figure 2. Inversement, une légère traction de la sonde orientera le microcâble vers l'apex du ventricule gauche 16.

Le praticien dispose ainsi d'une marge importante de contrôle de la direction de pénétration du microcâble 42 par rapport à la morphologie du ventricule gauche 16.

L'étape suivante consiste à percer le septum interventriculaire au moyen du microcâble.

Ce perçage peut être réalisé par une simple poussée mécanique du microcâble, si la rigidité de celui-ci est suffisante.

Mais dans une forme de mise en oeuvre préférentielle, ce perçage est assisté par une technique de ponction RF, que l'on va décrire ci-après.

Après avoir orienté le microcâble au contact de la paroi septale 20, ce microcâble est relié à un générateur de ponction RF en couplant la borne de sortie de celui-ci au connecteur électrique 52 relié à l'extrémité proximale 54 du microcâble 42 (connecteur qui, dans sa fonction normale, est destiné à être ultérieurement relié au boîtier du générateur de stimulation/défibrillation ou resynchronisation).

Le générateur de ponction RF 50 est un modèle connu, par exemple le *BMC Radio Frequency Perforation Generator* de Bayliss Medical Company, Inc. Ce générateur 50 est utilisé, en le couplant au microcâble 42, pour opérer une "ponction RF", technique consistant à appliquer localement une énergie radiofréquence produite par un générateur électronique RF approprié afin de créer une ouverture de très faible dimension dans le tissu cardiaque, plus précisément dans la paroi septale. Une telle ponction RF est opérée par application d'une énergie de faible puissance (5 à 25 W), pendant une brève période de temps (1 à 3 s) et sous haute tension (150 à 180 V), de manière à n'occasionner qu'un minimum de dommages collatéraux aux tissus environnants. Cette technique de "ponction RF" est à distinguer de l'"ablation RF", qui consiste à appliquer une puissance élevée (35 à 50 W) pendant une durée prolongée (60 à 90 secondes) et sous basse tension (35 à 50 V). L'ablation RF aurait pour effet, dans l'application considérée ici, de créer une lésion plus étendue, avec destruction thermique des tissus environnants.

L'énergie RF produite par le générateur de ponction RF 50 est appliquée à l'extrémité distale du microcâble 42, et cette énergie va permettre la réalisation d'une ponction très fine dans le septum interventriculaire 20, le diamètre de cette ponction étant défini par le diamètre du microcâble 42, par exemple de l'ordre de 0,5 à 0,7 mm.

Cette ponction est obtenue en combinant d'une part la fonction, obtenue par la sonde à vis, de guidage du microcâble, avec d'autre part l'application d'une énergie RF à ce microcâble pour le faire pénétrer dans le septum et minimiser ainsi considérablement l'effort axial à transmettre à l'extrémité du microcâble pendant cette étape. En effet, en l'absence d'énergie RF appliquée, le microcâble, trop fin, ne pourrait pas pénétrer dans les tissus et s'arc-bouterait au point de contact avec la paroi du septum. On notera par ailleurs que la technique de ponction RF assure une cautérisation des tissus traversés, donc empêche les saignements.

En résumé, la fonction de la sonde 28 est multiple :
- sélection du site de ponction du septum ;
- guidage du microcâble lors du trajet intraveineux ;
- transmission de la poussée jusqu'à l'extrémité du microcâble pendant la ponction ;
- stabilisation du microcâble pendant la ponction RF du septum ;
- définition (orientation) de la trajectoire du microcâble pendant la ponction RF, avec possibilité de tester plusieurs configurations d'implantation ;
- enfin, stabilisation et protection de la grande longueur du microcâble côté droit pendant toute la durée de vie du dispositif.

Après que la paroi 20 ait été complètement traversée, le générateur de ponction RF 50 est arrêté et déconnecté du microcâble.

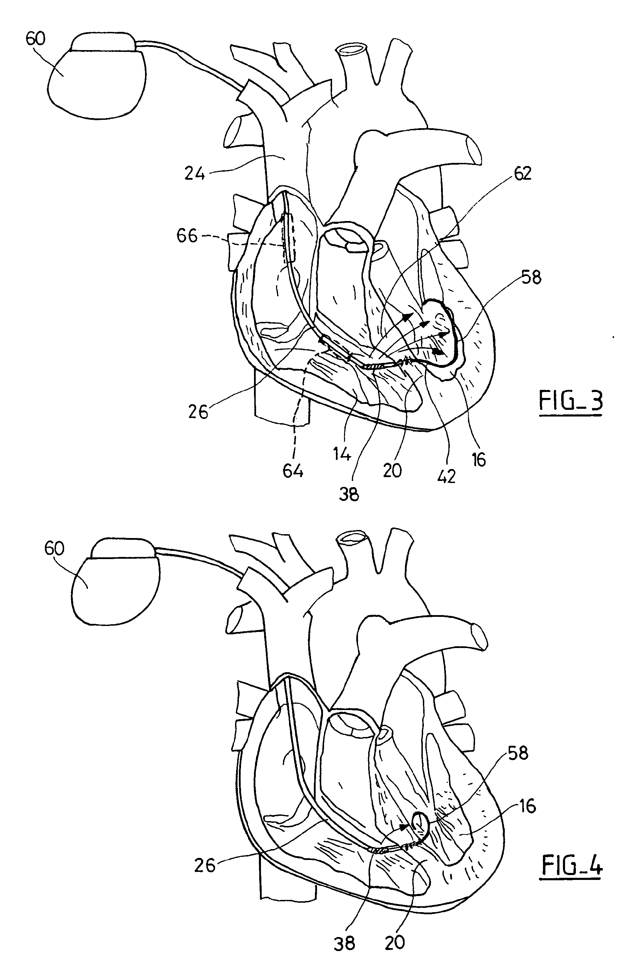

L'étape suivante, illustrée Figures 3 et 5, consiste à pousser le microcâble 42 au-delà du septum interventriculaire 20, dorénavant traversé de part en part, de manière à laisser émerger dans le volume intérieur du ventricule gauche 16, au-delà de la partie intermédiaire 56 enserrée dans le septum 20, une partie libre 58 dont la longueur peut aller jusqu'à 120 mm, avec dans l'exemple illustré Figure 3 une longueur d'environ 50 mm.

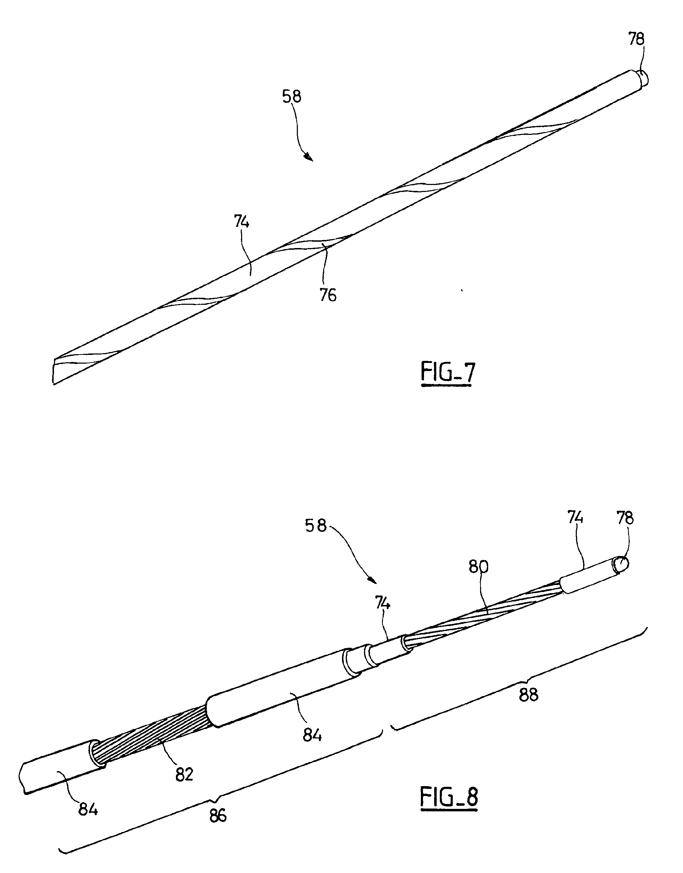

Cette partie libre émergente 58 du microcâble 42 totalement ou partiellement dénudée (on donnera des exemples plus bas en référence aux Figures 7 et 8), de manière à constituer une partie active, électriquement non isolée, susceptible de venir en contact en un ou plusieurs points avec la paroi du ventricule gauche 16.

L'étape suivante consiste à raccorder le générateur implanté 60 (stimulateur, défibrillateur et/ou resynchroniseur) à la sonde 26 et au microcâble 42, de sorte que l'une des bornes du circuit de détection/stimulation de ce générateur soit électriquement reliée à l'électrode distale 38 de la sonde 26 (électrode localisée dans le ventricule droit) et que l'autre borne de ce même circuit soit reliée à la partie libre active 58 du microcâble 42 (partie localisée dans le ventricule gauche).

L'application entre ces deux électrodes d'impulsions de stimulation produira un champ électrique, schématisé par les flèches 62, englobant une partie importante de la masse cardiaque et permettant donc une stimulation efficace du ventricule gauche. On notera que l'emplacement du site de stimulation semble beaucoup moins critique avec une stimulation endocavitaire que dans le cas d'une stimulation indirecte via le réseau coronaire. Concrètement, même si l'on maîtrise moins bien le site de stimulation (c'est-à-dire le point de contact de la partie active du microcâble 42 avec la paroi du ventricule gauche 16), on peut cependant s'attendre à une qualité de stimulation au moins aussi élevée que pour le meilleur site de stimulation indirecte via la réseau coronaire.

On notera qu'il est possible d'introduire de cette manière, via la même sonde 26, une pluralité de microcâbles tels que 42, qui émergeront chacun dans le ventricule gauche à partir du même site de ponction.

Dans le cas d'un défibrillateur, la partie libre active 58 du microcâble 42 est reliée à l'une des bornes du circuit de choc du générateur 60, et l'autre borne de ce circuit est reliée :
- à un bobinage 64 de type RV disposé dans le ventricule droit 14, et/ou
- à un bobinage 66 de type SVC disposé dans la veine cave supérieure 24 au voisinage de l'oreillette droite 12, et/ou
- au boîtier du générateur 60, et/ou
- à l'électrode 38 si celle-ci présente une surface suffisante (de l'ordre de 40 mm²).

On notera qu'elle telle configuration permet, avantageusement, d'englober une masse cardiaque maximale, en dépit de la surface d'électrode relativement réduite par rapport à une électrode de choc classique. En outre, l'application du choc se fera entre des électrodes situées de part et d'autre du septum, celui-ci étant pris "en tenaille" dans le champ électrique : ceci permet d'augmenter encore l'efficacité de la défibrillation, à énergie constante, ou bien de réduire notablement l'énergie du choc délivré et donc la douleur associée, par rapport à une configuration conventionnelle, où le choc serait délivré entre le boîtier du générateur 60 et les bobinages 64 et/ou 66.

La Figure 4 illustre une variante de la Figure 5, dans laquelle la partie active 58 du microcâble émergeant dans le ventricule gauche 16 présente une longueur beaucoup plus faible (de l'ordre de 10 mm ou moins) avec une forme de boucle, de manière à plaquer l'extrémité du microcâble contre le septum du ventricule gauche, définissant de ce fait un site de stimulation localisé de façon précise. La stimulation reste endocavitaire, mais la mobilité du microcâble et la surface de ce dernier exposée à la circulation artérielle sont très fortement réduites.

De façon générale, la technique que l'on vient de décrire présente de nombreux bénéfices :
- la microponction permet une réduction de section ponctionnée d'environ 90 % de la ponction par rapport à une approche transseptale interauriculaire classique, mettant en oeuvre un sonde de diamètre 5 French introduite dans un cathéter 7 French ;
- la taille de la ponction et la structure compacte du microcâble permettent de réaliser un passage au travers du septum interventriculaire - et non plus interauriculaire - en s'éloignant donc de la zone sensible de la crosse aortique ;
- pour les mêmes raisons, puisque le passage se fait au travers du septum interventriculaire, il n'est plus nécessaire de traverser la valve mitrale pour stimuler le ventricule gauche, par rapport à une approche transseptale interauriculaire classique : la solution proposée préserve donc intégralement le fonctionnement de cette valve ;
- diminution d'environ 75 % de la surface exposée à la circulation artérielle, par rapport à une approche transseptale interauriculaire classique, avec diminution corrélative du risque thrombo-embolique ;
- configuration compatible avec une extraction éventuelle, du fait du diamètre réduit et constant, et d'une résistance à la traction importante (d'environ 3 kg contre 1 kg pour une sonde classique) ;
- méthode de pose simplifiée, ne nécessitant qu'un matériel conventionnel ;
- stimulation conjointe du septum et de la paroi du ventricule gauche par une seule et même sonde ;
- très faible endommagement des tissus - à la différence d'une sonde endocavitaire gauche à vis, fortement traumatique notamment à l'endroit du site où l'on veut stimuler ;
- gestion simplifiée des risques d'embolie gazeuse : l'élément débouchant dans le ventricule gauche étant un composant massif (le microcâble) et non un cathéter creux, le système est moins sensible aux risques d'embolie gazeuse.

On va maintenant décrire un exemple de structure de microcâble convenant à la mise en oeuvre de l'invention, en référence aux Figures 6 à 8.

Le diamètre typique du coeur du microcâble, c'est-à-dire de la partie métallique non isolée de celui-ci, est de l'ordre de 0,2 à 0,4 mm. L'isolation de ce coeur est assurée par une gaine isolante de type polyuréthanne, silicone ou ETFE, conduisant à un diamètre total de l'ordre de 0,5 à 0,7 mm pour le microcâble avec sa gaine.

Ces dimensions sont à comparer au diamètre de l'ordre de 0,8 mm des aiguilles de ponction des kits transseptaux standards (perçage du septum auriculaire avec accès par la voie fémorale), et à celui des cathéters 7 French (2,33 mm) ou 9 French (3 mm) et des sondes 5 French (1,66 mm) ou 6 French (2 mm) utilisés dans les techniques classiques de pose d'une sonde endocavitaire gauche avec approche transseptale. Le microcâble de l'invention, même revêtu d'une gaine isolante, est donc d'un diamètre très inférieur à ce qui est usuellement utilisé pour les interventions transseptales.

Comme le microcâble traverse le septum dans sa partie intermédiaire 56, il doit pouvoir résister aux mouvements de compression dans cette région, et donc être particulièrement endurant à l'égard des sollicitations en cisaillement. Il doit être également très résistant aux sollicitations en flexion générées par le mouvement du ventricule gauche, qui vient solliciter à chaque battement la partie libre active 58 du microcâble.

On a illustré sur la Figure 6 un exemple de structure permettant de répondre à ces différentes exigences, en procurant en outre une radio-opacité (pour le suivi de l'intervention sous amplificateur de brillance) et également une conductivité suffisante pour garantir la délivrance des impulsions de stimulation ou de défibrillation avec un minimum de pertes en ligne par effet Joule.

Sur la structure illustrée Figure 6, le coeur du microcâble est constitué d'une pluralité de brins composites 68 toronnés ensemble, avec un brin central entouré de six brins périphériques. Chaque brin composite 68 est lui-même constitué d'un fil d'âme central 70 en platine iridié (pour la radio-opacité) entouré d'une pluralité de fils également composites 72 procurant les propriétés mécaniques et électriques recherchées, par exemple six fils 72. Chaque fil 72 est par exemple constitué d'un coeur d'argent (pour la conductivité électrique) enveloppé de nitinol (pour les propriétés de résistance aux contraintes mécaniques). Ces différents fils sont disponibles dans le commerce, par exemple auprès de la Société Fort Wayne Metals Inc., Fort Wayne, USA, et sont utilisés dans le domaine médical notamment pour réaliser des conducteurs de défibrillation.

La structure illustrée n'est en aucune façon limitative, et de nombreuses configurations de microcâbles peuvent être envisagées, par exemple des structures de type "1x3" (microcâble constitué d'un toron de 3 fils), 1x7, 1x19, 7x7 (comme dans l'exemple illustré figure 6, où le microcâble est formé de 7 torons de 7 fils chacun), 7x19, 19x7, 7x7x7, etc.

Les avantages d'une telle structure résident dans le fait que les éléments les moins endurants mécaniquement (platine iridié et argent) sont encapsulés soit directement dans la gaine en nitinol (pour l'argent), soit au coeur de chaque toron (pour le platine iridié). Les conséquences d'une fracture éventuelle de ces brins sont donc minimisées.

L'isolant externe 74 est par exemple une gaine de type polyuréthanne, silicone ou ETFE, qui assure une encapsulation supplémentaire du toron du microcâble.

La partie libre active 58 du microcâble est au moins partiellement dénudée. De préférence, la surface totale de la (des) région(s) dénudée(s) de cette partie libre active est comprise entre 4 et 6 mm², valeur du même ordre que la surface active d'une sonde endocavitaire standard.

Dans la mesure où l'extrémité libre 58 n'est pas ancrée dans la paroi du ventricule gauche, il convient de maximiser les chances de contact de la région dénudée du microcâble avec les zones cibles de la paroi du ventricule.

Une première possibilité, illustrée Figure 7, consiste à dénuder l'isolant 74 le long d'une découpe hélicoïdale unique 76 courant sur tout ou partie de la longueur de la région active. L'extrémité distale 78 est également laissée dénudée, principalement pour assurer la sortie des courants de ponction RF, et elle est conformée de manière à rester atraumatique. Cette solution permet d"'allonger" la surface de stimulation sans en augmenter la surface totale, et donc d'augmenter la probabilité de contact avec les tissus de la cavité ventriculaire gauche ; dans la situation la plus défavorable, au moins une portion du microcâble reste en contact avec la paroi, assurant ainsi une distance maximale de quelques dixièmes de millimètres entre la surface dénudée et les tissus à stimuler.

En variante ou en complément, les zones conductrices dénudées du microcâble peuvent recevoir un revêtement de type poreux, par exemple de type TiNi, ou être encapsulées par un composant uniformément serti par une technique de *swaging* (matriçage), lui-même revêtu afin d'améliorer les performances électriques de l'ensemble. Il est également possible de prévoir une couche additionnelle formée par un film de carbone déposé par pulvérisation cathodique, pour améliorer les propriétés de biocompatibilité entre le microcâble, son isolant et son environnement, de manière à éviter toute dégradation des parties en contact avec un flux sanguin.

Les US-A-5 370 684 et 5 387 247, tous deux au nom de Sorin Biomedica SpA, décrivent la manière de déposer par pulvérisation cathodique un film mince, submicronique, de carbone sur des prothèses implantables telles que cathéters, valves cardiaques, etc. en polyuréthanne ou silicone.. On se réfèrera à ces deux documents pour plus de détails sur la technologie permettant de réaliser ce dépôt.

Une autre possibilité, illustrée Figure 8, consiste à prévoir sur le microcâble une pluralité de régions dénudées distinctes 80, 82, s'étendant en succession le long de la partie libre active 58.

De plus, dans la variante illustrée Figure 8, le microcâble a été pourvu, dans la partie active 58 à l'exception de l'extrémité distale, d'un manchon raidisseur permettant de faire varier la raideur de l'ensemble le long de la partie émergente. Le manchon 82 est par exemple un tube de type HHS (marque déposée) de Fort Wayne Metals Inc., Fort Wayne, USA, qui est un tube creux formé de brins hélicoïdaux de flexibilité contrôlée conçus à cet effet.

Le tube 82 est partiellement revêtu d'un isolant 84, de manière à définir une structure bipolaire (régions dénudées 80, 82), voire multipolaire. Cette configuration permet de localiser plus précisément le champ électrique, par exemple pour garantir lors de la stimulation une onde de dépolarisation plus rapide de l'endocarde vers l'épicarde.

On notera que la structure reste compacte, non creuse, et résistante aux sollicitations de compression générées par la contraction du septum. Le corps plus raide 86 (au niveau du tube raidisseur 82) permet de projeter l'extrémité distale atraumatique plus souple 88 contre la paroi libre du ventricule gauche, garantissant ainsi un meilleur contact des zones conductrices du microcâble avec les tissus.

## Revendications

1. Un ensemble de stimulation/défibrillation endocavitaire du ventricule gauche, comprenant :
- un boîtier (60) de stimulateur, défibrillateur et/ou resynchroniseur implantable, comprenant des moyens générateurs aptes à délivrer entre deux bornes des impulsions électriques de stimulation/défibrillation ; et
- une sonde endocavitaire comprenant côté distal au moins une électrodes de stimulation/défibrillation couplées côté proximal à une borne correspondante des moyens générateurs,
dans lequel la sonde comprend :
- un corps de sonde (26) en matériau déformable avec une lumière interne ouverte côté distal, l'extrémité distale (30) du corps de sonde étant apte à s'étendre dans la cavité du ventricule droit (14) et étant pourvue de moyens (32) d'ancrage à la paroi du septum interventriculaire (20), le corps de sonde portant au moins une électrode (38 ; 64, 66) de stimulation/défibrillation couplée côté proximal à une première des deux bornes des moyens générateurs ; et
- au moins une structure apte à être déployée dans la cavité du ventricule gauche, comprenant un coeur électriquement conducteur (70, 72) protégé par une gaine isolante (74), cette structure étant logée et apte à coulisser dans la lumière du corps de sonde en s'étendant sur toute la longueur du corps de sonde et au-delà de l'extrémité distale de celui-ci, la partie située au-delà de l'extrémité distale du corps de sonde comprenant successivement :
• une partie intermédiaire (56), apte à traverser de part en part la paroi du septum interventriculaire (20), et
• une partie libre active (58), apte à émerger dans la cavité du ventricule gauche (16), cette partie libre comprenant au moins une région dénudée (76 ; 80, 82),
ladite structure étant électriquement couplée à son extrémité proximale (54) à la seconde des deux bornes des moyens générateurs,
de manière à produire, lors de l'activation des moyens générateurs, un champ électrique (62) entre, d'une part, ladite au moins une électrode (38 ; 64, 66) de stimulation/défibrillation du corps de sonde et, d'autre part, ladite au moins une région dénudée (76 ; 80, 82) de la partie libre active (58) de ladite structure,
**caractérisé en ce que** ladite structure apte à être déployée dans la cavité du ventricule gauche est un microcâble (42) présentant un diamètre de coeur électriquement conducteur compris, au moins dans la partie libre active, entre 0,2 et 0,4 mm,
et **en ce que** ce microcâble comporte un toron formé d'une pluralité de brins avec au moins un brin composite (68) comprenant :
- un premier matériau présentant des propriétés de résistance mécanique à la fatigue en flexion supérieures à celles des autres matériaux du brin ; et
- un deuxième matériau radio-opaque.

2. L'ensemble de la revendication 1, dans lequel la longueur de la partie libre active (58) est comprise entre 30 et 120 mm, de préférence 45 à 55 mm.

3. L'ensemble de la revendication 1, dans lequel la longueur de la partie intermédiaire (56) est comprise entre 10 et 20 mm.

4. L'ensemble de la revendication 1, dans lequel le diamètre de la gaine isolante du microcâble est compris entre 0,5 et 0,7 mm.

5. L'ensemble de la revendication 1, dans lequel ledit au moins un brin composite (68) comprend en outre :
- un troisième matériau présentant des propriétés de conductivité électrique supérieures à celles des autres matériaux du brin.

6. L'ensemble de la revendication 1, dans lequel le toron du microcâble est formé d'une pluralité de brins composites (68), avec un fil d'âme (70) comprenant ledit deuxième matériau, ce fil d'âme étant entouré par des fils composites (72) comprenant chacun ledit premier matériau comme matériau de surface.

7. L'ensemble de la revendication 1, dans lequel la partie libre active (58) du microcâble comporte une pluralité de régions dénudées distinctes (80, 82) s'étendant en succession le long de cette partie libre active.

8. L'ensemble de la revendication 1, dans lequel la partie libre active (58) du microcâble est pourvue, à l'exception de l'extrémité distale, d'un manchon raidisseur (82) apte à faire varier la raideur de l'ensemble le long de la partie émergente.

9. L'ensemble de la revendication 8, dans lequel le manchon raidisseur (82) est un tube creux formé de brins hélicoïdaux de flexibilité contrôlée et partiellement revêtu d'un isolant (84), de manière à définir une structure bipolaire ou multipolaire.

10. L'ensemble de la revendication 1, dans lequel la partie libre active (58) du microcâble comporte une région dénudée hélicoïdale (76) s'étendant le long de cette partie libre active.

11. L'ensemble de la revendication 1, dans lequel la surface totale de la (des) région(s) dénudée(s) (76 ; 80, 82) de la partie libre active (58) du microcâble est d'au plus 40 mm², de préférence comprise entre 4 et 6 mm².

12. L'ensemble de la revendication 1, comprenant également :
- un générateur de ponction radiofréquence (50), apte à être relié au microcâble pour permettre l'application contrôlée à l'extrémité distale de celui-ci d'une énergie radiofréquence pour réaliser une traversée de la paroi du septum par ce microcâble.

13. L'ensemble de la revendication 1, dans lequel les moyens d'ancrage de l'extrémité distale du corps de sonde comprennent une vis hélicoïdale saillante (32) apte à pénétrer dans la paroi du septum interventriculaire (20) sous l'effet d'un mouvement de vissage imprimé au corps de sonde (26) depuis l'extrémité proximale celui-ci.

14. L'ensemble de la revendication 1, dans lequel ladite électrode du corps de sonde couplée à ladite première borne des moyens générateurs est une électrode de stimulation (38), disposée dans la région distale du corps de sonde.

15. L'ensemble de la revendication 1, dans lequel dans lequel ladite électrode du corps de sonde couplée à ladite première borne des moyens générateurs est un bobinage formant électrode de défibrillation (64, 66), de type RV apte à être disposé dans le ventricule droit ou de type SVC apte à être disposé dans la veine cave supérieure au voisinage de l'oreillette.

16. L'ensemble de la revendication 1, dans lequel la partie libre active du microcâble comprend une partie d'extrémité distale (88) présentant une raideur en flexion inférieure à celle d'une partie attenante moins distale (86).

17. L'ensemble de la revendication 1, dans lequel la partie libre active est comprise entre 5 et 15 mm et est conformée en une boucle plate.

## Claims

1. Endocavitary stimulation/defibrillation assembly for the left ventricle, comprising:
- an implantable stimulator, defibrillator and/or resynchronizer module (60), comprising generator means capable of delivering, between two terminals, electrical stimulation/defibrillation pulses; and
- an endocavitary lead comprising, at the distal end, at least one stimulation/defibrillation electrode coupled, at the proximal end, to a corresponding terminal of the generator means,
in which the lead comprises:
- a lead body (26) made of a deformable material with an open internal light at the distal end, the distal end (30) of the lead body being capable of extending into the cavity of the right ventricle (14) and being provided with means (32) for anchoring to the wall of the interventricular septum (20), the lead body bearing at least one stimulation/defibrillation electrode (38; 64, 66) coupled at the proximal end to a first of the two terminals of the generator means; and
- at least one structure capable of being deployed into the cavity of the left ventricle, comprising an electrically conductive core (70, 72) protected by an insulating sheath (74), this structure being housed and being capable of sliding in the light of the lead body by extending over the entire length of the lead body and beyond the distal end thereof, the portion situated beyond the distal end of the lead body comprising, in succession:
• an intermediate portion (56), capable of passing right through the wall of the interventricular septum (20), and
• an active free portion (58), capable of emerging into the cavity of the left ventricle (16), this free portion comprising at least one stripped region (76; 80, 82),
said structure being electrically coupled at its proximal end (54) to the second of the two terminals of the generator means,
so as to produce, upon activation of the generator means, an electrical field (62) between, on the one hand, said at least one stimulation/defibrillation electrode (38; 64, 66) of the lead body and, on the other hand, said at least one stripped region (76; 80, 82) of the active free portion (58) of said structure,
**characterized in that** said structure capable of being deployed into the cavity of the left ventricle is a microcable (42) having an electrically conductive core diameter of between, at least in the active free portion, 0.2 and 0.4 mm,
and **in that** this microcable includes a bundle formed by a plurality of strands with at least one composite strand (68) comprising:
- a first material having properties of mechanical resistance to flexural fatigue greater than those of the other materials of the strand; and
- a second, radio-opaque material.

2. Assembly according to Claim 1, in which the length of the active free portion (58) is between 30 and 120 mm, preferably 45 to 55 mm.

3. Assembly according to Claim 1, in which the length of the intermediate portion (56) is between 10 and 20 mm.

4. Assembly according to Claim 1, in which the diameter of the insulating sheath of the microcable is between 0.5 and 0.7 mm.

5. Assembly according to Claim 1, in which said at least one composite strand (68) also comprises:
- a third material having electrical conductivity properties greater than those of the other materials of the strand.

6. Assembly according to Claim 1, in which the bundle of the microcable is formed by a plurality of composite strands (68), with a core wire (70) comprising said second material, this core wire being surrounded by composite wires (72) each comprising said first material as surface material.

7. Assembly according to Claim 1, in which the active free portion (58) of the microcable includes a plurality of distinct stripped regions (80, 82) extending in succession along this active free portion.

8. Assembly according to Claim 1, in which the active free portion (58) of the microcable is provided, except for the distal end, with a stiffening shroud (82) capable of varying the stiffness of the assembly along the emergent portion.

9. Assembly according to Claim 8, in which the stiffening shroud (82) is a hollow tube formed by helical strands of controlled flexibility and partially coated with an insulator (84), so as to define a bipolar or multipolar structure.

10. Assembly according to Claim 1, in which the active free portion (58) of the microcable includes a helical stripped region (76) extending along this active free portion.

11. Assembly according to Claim 1, in which the total surface area of the stripped region(s) (76; 80, 82) of the active free portion (58) of the microcable is at most 40 mm², preferably between 4 and 6 mm².

12. Assembly according to Claim 1, also comprising:
- a radiofrequency puncture generator (50), capable of being linked to the microcable to allow for the controlled application at the distal end thereof of a radiofrequency energy in order to pass this microcable through the wall of the septum.

13. Assembly according to Claim 1, in which the anchoring means of the distal end of the lead body comprise a protruding helical screw (32) capable of penetrating into the wall of the interventricular septum (20) under the effect of a screwing motion imparted on the lead body (26) from the proximal end thereof.

14. Assembly according to Claim 1, in which said electrode of the lead body coupled to said first terminal of the generator means is a stimulation electrode (38), arranged in the distal region of the lead body.

15. Assembly according to Claim 1, in which said electrode of the lead body coupled to said first terminal of the generator means is a winding forming a defibrillation electrode (64, 66), of RV type capable of being arranged in the right ventricle or of SVC type capable of being arranged in the superior vena cava in the vicinity of the auricle.

16. Assembly according to Claim 1, in which the active free portion of the microcable comprises a distal end portion (88) having a flexural stiffness less than that of a less distal adjoining portion (86).

17. Assembly according to Claim 1, in which the active free portion is between 5 and 15 mm and is configured in a flat loop.

## Patentansprüche

1. Einheit zur endokavitären Stimulation/Defibrillation des linken Ventrikels, die Folgendes enthält:
- ein implantierbares Stimulator-, Defibrillator - und/oder Resynchronisierergehäuse (60), das Generatoreinrichtungen enthält, die zwischen zwei Anschlüssen elektrische Stimulations- /Defibrillationsimpulse liefern können; und
- eine endokavitäre Sonde, die auf der distalen Seite mindestens eine Stimulations-/Defibrillationselektrode enthält, die auf der proximalen Seite mit einem entsprechenden Anschluss der Generatoreinrichtungen gekoppelt ist,
wobei die Sonde Folgendes enthält:
- einen Sondenkörper (26) aus verformbarem Material mit einem auf der distalen Seite offenen inneren Kanal, wobei das distale Ende (30) des Sondenkörpers sich in den Hohlraum des rechten Ventrikels (14) erstrecken kann und mit Einrichtungen (32) zur Verankerung an der Wand des interventrikulären Septums (20) versehen ist, wobei der Sondenkörper mindestens eine Stimulations-/Defibrillationselektrode (38; 64, 66) trägt, die auf der proximalen Seite mit einem ersten der zwei Anschlüsse der Generatoreinrichtungen gekoppelt ist; und
- mindestens eine Struktur, die im Hohlraum des linken Ventrikels ausgebreitet werden kann, die einen elektrisch leitenden Kern (70, 72) enthält, der von einer Isolierhülle (74) geschützt wird, wobei diese Struktur in dem Kanal des Sondenkörpers untergebracht und in der Lage ist, darin zu gleiten, indem sie sich über die ganze Länge des Sondenkörpers und über dessen distales Ende hinaus erstreckt, wobei der sich jenseits des distalen Endes des Sondenkörpers befindliche Teil nacheinander Folgendes enthält:
• einen Zwischenteil (56), der die Wand des interventrikulären Septums (20) von einer Seite zur anderen durchqueren kann, und
• einen aktiven freien Teil (58), der im Hohlraum des linken Ventrikels (16) hervortreten kann, wobei dieser freie Teil mindestens einen abisolierten Bereich (76; 80, 82) enthält,
wobei die Struktur an ihrem proximalen Ende (54) mit dem zweiten der zwei Anschlüsse der Generatoreinrichtungen elektrisch gekoppelt ist, um bei der Aktivierung der Generatoreinrichtungen zwischen einerseits der mindestens einen Stimulations-/Defibrillationselektrode (38; 64, 66) des Sondenkörpers und andererseits dem mindestens einen abisolierten Bereich (76; 80, 82) des aktiven freien Teils (58) der Struktur ein elektrisches Feld (62) zu erzeugen,
**dadurch gekennzeichnet, dass** die Struktur, die im Hohlraum des linken Ventrikels ausgebreitet werden kann, ein Mikrokabel (42) ist, das einen Durchmesser des elektrisch leitenden Kerns aufweist, der zumindest im aktiven freien Teil zwischen 0,2 und 0,4 mm liegt,
und dass dieses Mikrokabel eine Litze aufweist, die von einer Vielzahl von Einzelsträngen geformt wird, mit mindestens einem Verbund-Einzelstrang (68), der Folgendes enthält:
- ein erstes Material, das höhere mechanische Festigkeitseigenschaften gegenüber der Biegeermüdung als die der anderen Materialien des Einzelstrangs hat; und
- ein zweites, röntgenstrahlenundurchlässiges Material.

2. Einheit nach Anspruch 1, bei der die Länge des aktiven freien Teils (58) zwischen 30 und 120 mm, vorzugsweise zwischen 45 und 55 mm, liegt.

3. Einheit nach Anspruch 1, bei der die Länge des Zwischenteils (56) zwischen 10 und 20 mm liegt.

4. Einheit nach Anspruch 1, bei der der Durchmesser der Isolierhülle des Mikrokabels zwischen 0,5 und 0,7 mm liegt.

5. Einheit nach Anspruch 1, bei der der mindestens eine Verbund-Einzelstrang (68) außerdem Folgendes enthält:
- ein drittes Material, das höhere elektrische Leitfähigkeitseigenschaften als die anderen Materialien des Einzelstrangs aufweist.

6. Einheit nach Anspruch 1, bei der die Litze des Mikrokabels von einer Vielzahl von Verbund-Einzelsträngen (68) geformt wird, mit einem das zweite Material enthaltenden Kerndraht (70), wobei dieser Kerndraht von Verbunddrähten (72) umgeben ist, die jeder das erste Material als Oberflächenmaterial enthalten.

7. Einheit nach Anspruch 1, bei der der aktive freie Teil (58) des Mikrokabels eine Vielzahl von unterschiedlichen abisolierten Bereichen (80, 82) aufweist, die sich aufeinanderfolgend entlang diesem aktiven freien Teil erstrecken.

8. Einheit nach Anspruch 1, bei der der aktive freie Teil (58) des Mikrokabels mit Ausnahme des distalen Endes mit einer Versteifungsmanschette (82) versehen ist, die die Steifheit der Einheit entlang dem hervorstehenden Teil variieren lassen kann.

9. Einheit nach Anspruch 8, bei der die Versteifungsmanschette (82) ein Hohlrohr ist, das von spiralförmigen Einzelsträngen kontrollierter Flexibilität geformt und teilweise mit einer Isolierung (84) verkleidet ist, um eine bipolare oder multipolare Struktur zu definieren.

10. Einheit nach Anspruch 1, bei der der aktive freie Teil (58) des Mikrokabels einen abisolierten spiralförmigen Bereich (76) aufweist, der sich entlang diesem aktiven freien Teil erstreckt.

11. Einheit nach Anspruch 1, bei der die Gesamtfläche des (der) abisolierten Bereichs (Bereiche) (76; 80, 82) des aktiven freien Teils (58) des Mikrokabels höchstens 40 mm² beträgt, vorzugsweise zwischen 4 und 6 mm² liegt.

12. Einheit nach Anspruch 1, die ebenfalls Folgendes enthält:
- einen Hochfrequenz-Punktionsgenerator (50), der mit dem Mikrokabel verbunden werden kann, um das kontrollierte Anlegen einer Hochfrequenzenergie an dessen distales Ende zu erlauben, um eine Durchquerung der Wand des Septums durch dieses Mikrokabel durchzuführen.

13. Einheit nach Anspruch 1, bei der die Einrichtungen zur Verankerung des distalen Endes des Sondenkörpers eine vorstehende Spiralschraube (32) enthalten, die unter der Wirkung einer dem Sondenkörper (26) ausgehend von dessen proximalem Ende verliehenen Schraubbewegung in die Wand des interventrikulären Septums (20) eindringen kann.

14. Einheit nach Anspruch 1, bei der die mit dem ersten Anschluss der Generatoreinrichtungen gekoppelte Elektrode des Sondenkörpers eine Stimulationselektrode (38) ist, die im distalen Bereich des Sondenkörpers angeordnet ist.

15. Einheit nach Anspruch 1, bei der die mit dem ersten Anschluss der Generatoreinrichtungen gekoppelte Elektrode des Sondenkörpers eine Wicklung ist, die eine Defibrillationselektrode (64, 66) vom Typ RV, die im rechten Ventrikel angeordnet werden kann, oder vom Typ SVC bildet, die in der oberen Hohlvene in der Nähe des Vorhofs angeordnet werden kann.

16. Einheit nach Anspruch 1, bei der der aktive freie Teil des Mikrokabels einen distalen Endteil (88) enthält, der eine geringere Biegesteifigkeit hat als diejenige eines weniger distalen angrenzenden Bereichs (86).

17. Einheit nach Anspruch 1, bei der der aktive freie Teil zwischen 5 und 15 mm liegt und als eine flache Schleife geformt ist.
